# EUROPÄISCHE PATENTSCHRIFT

(11) **EP 2 662 080 B1**
(45) Veröffentlichungstag und Bekanntmachung des Hinweises auf die Patenterteilung: **24.02.2016**
(21) Anmeldenummer: 11870136.6
(22) Anmeldetag: 26.07.2011
(51) Int. Cl.: A61K 31/195, A61K 33/44, B82B 1/00, B82Y 5/00, A61K 47/48

(54) **NANODIAMANTKONJUGAT MIT GLYCIN UND VERFAHREN ZUR HERSTELLUNG DIESES KONJUGATS**
NANO-DIAMOND CONJUGATE WITH GLYCINE AND METHOD FOR PRODUCING SAID CONJUGATE
CONJUGUÉ DE NANO-DIAMANT ET DE GLYCINE ET PROCÉDÉ DE SA FABRICATION

(43) Veröffentlichungstag der Anmeldung: 13.11.2013
(73) Patentinhaber: Zakrytoe Aktsionernoe Obschestvo "Almaz Pharm", Moscow 117393 (RU)
(72) Erfinder: YAKOVLEV, Ruslan Jur'evich, 300001 Tula (RU)
(74) Vertreter: Jeck, Anton
(86) Internationale Anmeldenummer: PCT/RU2011/000490
(87) Internationale Veröffentlichungsnummer: WO 2013/015702

(56) Entgegenhaltungen:
- US-B2- 7 820 130
- US-B2- 7 820 130
- LISICHKIN G V ET AL: "Halogenation of detonation-synthesised nanodiamond surfaces", MENDELEEV COMMUNICATIONS, INSTITUTE OF PHYSICS PUBLISHING, BRISTOL, GB, Bd. 19, Nr. 6, 1. November 2009 (2009-11-01), Seiten 309-310, XP026770054, ISSN: 0959-9436 [gefunden am 2009-11-20]
- ANDO ET AL: "Chemical modification of diamond surfaces using a chlorinated surface as an intermediate state", DIAMOND AND RELATED MATERIALS, ELSEVIER SCIENCE PUBLISHERS, AMSTERDAM, NL, Bd. 5, Nr. 10, 1. August 1996 (1996-08-01) , Seiten 1136-1142, XP005464376, ISSN: 0925-9635, DOI: 10.1016/0925-9635(96)00529-8
- KULAKOVA I I ET AL: "The structure of chemically modified detonation-synthesized nanodiamond particles", NANOTECHNOLOGIES IN RUSSIA, MAIK NAUKA - INTERPERIODICA, RU, Bd. 5, Nr. 7-8, 1. August 2010 (2010-08-01), Seiten 474-485, XP008168000, ISSN: 1995-0780, DOI: 10.1134/S1995078010070074
- LISICHKIN, GEORGII V.: 'Halogenation of detonation-synthesised nanodiamond surfaces' MENDELEEV COMMUN. vol. 19, 2009, pages 309 - 310, XP026770054
- KULAKOVA I.I. ET AL.: 'Stroenie chastits khmicheski modifitsirovannogo nanoalmaza detonatsionnnogo sinteza' ROSSIISKIE NANOTEKHNOLOGII 1992-7223 vol. 5, no. 7-8, 2010, pages 66 - 73, XP008168000

## Beschreibung

Die Erfindung betrifft ein Konjugat von Nanodiamanten mit Glyzin und ein Verfahren zur Herstellung des Konjugats.

Die Erfindung ist auf dem Gebiet der Pharmazie und der Medizin, nämlich auf dem Gebiet der pharmazeutischen Nanotechnologie, einsetzbar und betrifft ein Konjugat von Nanodiamanten mit Glyzin zur Förderung von Glyzin in den Körper. Sie betrifft auch ein Verfahren zur Herstellung dieses Konjugats.

Aus dem Stand der Technik ist ein Konjugat von Nanodiamanten mit Glyzin bekannt, welches aus Teilchen mit einer Korngröße von 2 - 10 nm besteht. Es wird als Bindemittel in Polymerzusammensetzungen angewendet [1, 2]. Die Besonderheit dieses Konjugats ist das Vorhandensein von Fluoratomen auf der Oberfläche der Nanodimantenteilchen. Der Anteil von Fluoratomen auf der Oberfläche der Nanodiamantenteilchen liegt unter 1 % (at.). Jedoch kann der Fluorgehalt in der Praxis 14 % (at.) und sogar mehr erreichen.

Im Stand der Technik ist auch ein Verfahren zur Herstellung von Konjugat von Nanodiamantenteilchen mit Glyzin beschrieben, welches als Bindemittel bei Epoxidharzmassen eingesetzt wird. Diese Verbundstoffe werden für Korrosionsschutzüberzüge verwendet. Das Verfahren wird wie folgt ausgeführt [2]: Eine Nanodiamanten-Einwaage wird in einen Reaktor mit einer konstanten Heliumströmung eingesetzt und bei einer Temperatur von 150 - 470° C im Laufe von 3 - 4 Stunden ausgeglüht. Danach werden die Proben der Nanodiamantenteilchen bei einer Temperatur von 50 - 500° C innerhalb von 1 - 24 Stunden fluoriert, indem sie mit einem Gemisch von Fluor- und Wasserstoffgasen kontaktieren. Um das Konjugat der Nanodiamantenteilchen mit Glyzin zu bekommen, wird der fluorierte Nanodiamant mit Ultraschall im *o*-Dichlorbenzol innerhalb von 20 - 30 Minuten behandelt, mit Glyzin-Äthyläthen-Hydrochlorid (NH₂CH₂COOCH₂CH₃·HCl) und mit einigen Tropfen von Pyridin ergänzt. Die resultierende Mischung wird bei einer Temperatur von 130 - 140° C im Laufe von 8 - 12 Stunden vermengt. Das produzierte Konjugat wird gefiltert, mit Äthanol gewaschen und im Vakuum bei 70° C getrocknet.

Es ist jedoch davon abzuraten, Konjugate der fluormodifizierten Nanodiamantenteilchen mit Glyzin für medizinische Zwecke zu nutzen, denn es ist bekannt, dass das in einem organischen Stoff vorhandene Fluor sowie seine Derivate seine Giftigkeit steigern. Es kann auch die Kennziffern des mikrosomalen Systems bei biologischer Transformation der Xenobiotika in der Leber beeinträchtigen [3]. Darüber hinaus neigen Fluor und seine Verbindungen dazu, sich in verschiedenen Objekten der Umwelt anzusammeln und darin in verschiedenen Mengen zu bleiben [4].

Gemäß der Erfindung wird ein Konjugat von Nanodiamantenteilchen mit Glyzin zur Förderung von Glyzin in den Körper beschrieben. Es stellt glyzinmodifizierte Nanodiamantenteilchen mit einer Korngröße von 2 - 10 nm dar. Diese Teilchen enthalten Glyzin bis zu 21 ± 3 % (Gew.), welches ein Bestandteil einer bis zu 1 nm starken Oberflächenhülle des Konjugats ist.

Das Konjugat von Nanodiamanten enthält ebenfalls keine Fluoratome auf seiner Oberfläche.

Der Glyzinanteil im Konjugat von Nanodiamantenteilchen mit Glyzin wird wie folgt festgestellt: Es wird eine Mischung von Nanodiamantenteilchen mit verschiedenen Glyzinmengen zubereitet. Jeder Mischung und der zu ermittelnden Probe wird je eine Einwaage gleicher Masse entnommen. Ihre IR-Spektren werden erfasst. Aus diesen IR-Spektren werden die stärksten Kennsignale ausgewählt, welche dann mit den Streifen des IR-Spektrums von Ausgangsglyzin verglichen werden. Danach werden Eichlinien ermittelt, um die Abhängigkeit der Signalstärke im IR-Spektrum vom Glyzingehalt in der Einwaage zu kennzeichnen. Dann wird der Glyzinanteil im Konjugat von Nanodiamantenteilchen mit Glyzin anhand der Eichlinien je nach Stärke der ausgewählten Kennstreifen des erforschten Konjugats von Nanodiamantenteilchen mit Glyzin bestimmt. Der Mittelanteil von Glyzin in einem Konjugat von Nanodiamantenteilchen mit Glyzin wird mit Hilfe der erworbenen Daten berechnet.

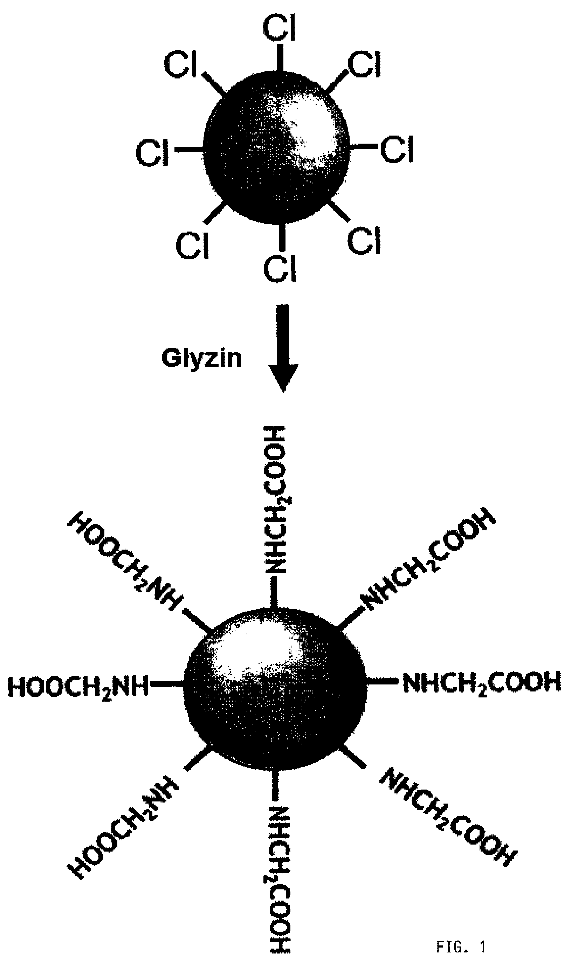

Gemäß der Erfindung wird auch ein Verfahren zur Herstellung von Konjugat von Nanodiamantenteilchen mit Glyzin mit einer Korngröße von 2 - 10 nm und einem Glyzingehalt bis zu 21 ± 3 % (Gew.) beschrieben (schematische Darstellung der Ausführung des Verfahrens ist Fig. 1 zu entnehmen). Die Nanodiamantenteilchen weisen dabei eine bis zu 1 nm starke Oberflächenhülle auf. Das Wesen des Verfahrens: Die chlormodifizierten Nanodiamantenteilchen mit einer Korngröße von 2 - 110 nm werden in einem Polar-Lösungsmittel aufgelöst, so dass eine Aufschwemmung entsteht. Danach werden ein tertiäres Amin und Glyzin zugesetzt. Das hergestellte Gemisch wird mit Ultraschall behandelt und nachfolgend bei einer Temperatur von 50 - 80° C gehalten, zentrifugiert, mit einem Lösungsmittel gewaschen und getrocknet.

Als tertiäres Amin wird Triäthylamin verwendet. Als Polar-Lösungsmittel werden Pyridin, niedere aliphatische Alkohole, ein Alkohol-Wasser-Gemisch oder Wasser verwendet. Die Ultraschallbehandlung wird im Laufe von 5 - 60 Min. durchgeführt. Das Halten bei einer Temperatur von 50 - 80° C erfolgt im Laufe von 18 bis 48 Stunden. Somit ist die Herstellung von fluorfreiem hochdispersem Konjugat von Nanodiamantenteilchen mit Glyzin sowie die Verminderung der Umwelt- und der endoökologischen Gefährdung, eine Vereinfachung und eine Kostensenkung des Verfahrens zur Herstellung von Konjugat von Nanodiamantenteilchen mit Glyzin eine vordringliche und praxisbezogene Aufgabe.

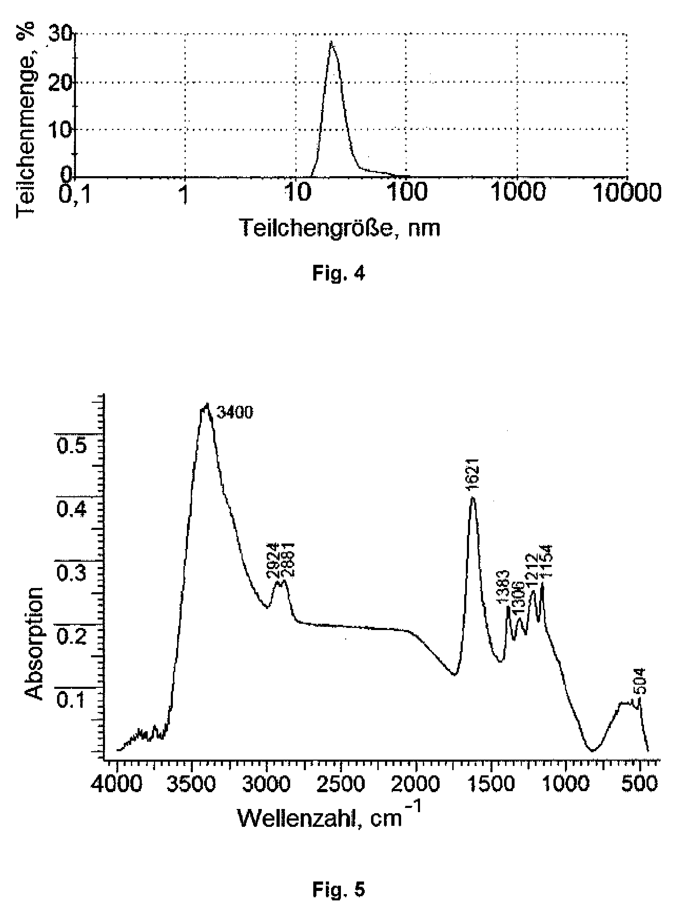

Das beschriebene Konjugat von Nanodiamantenteilchen mit einer Aminosäure Glyzin enthält keine Fluoratome auf seiner Oberfläche und stellt ein ultradisperses Pulver dar. Das Pulver ist dunkelgrau oder dunkelgrau mit dunkelblauer oder grüner Anfärbung. Die Teilchengröße liegt im Bereich von 2 - 10 nm, und die Aggregatgröße variiert von 25 - 50 nm in der wässrigen Suspension (Fig. 4).

Die Mikroaufnahme der Teilchen des Konjugats von Nanodiamantenteilchen mit Glyzin wurde mit Hilfe von einem elektronischen höchstauflösenden Abtast-Feldemissionsmikroskop Zeiss Ultra Plus (Carl Zeiss, Deutschland) gemacht. Die Aufnahmebedingungen sind der Mikroaufnahme zu entnehmen.

Die Mikroaufnahme der Teilchen des Konjugats von Nanodiamantenteilchen mit Glyzin wurde mit einem Durchstrahlungselektronenmikroskop Jeol 101 1 (JEOL, Japan) gemacht.

In Fig. 4. ist eine Verteilkurve des beschriebenen Konjugats von Nanodiamantenteilchen mit Glyzin in der Suspension dargestellt. Daraus folgt, dass die Teilchengrößen in der Suspension 25 - 50 nm betragen. Die Teilchengrößenverteilung in der Konjugat-Suspension wurde nach einem Verfahren der dynamischen Laserlichtstreuung mit Hilfe eines Geräts ZetaSizer (Malvern Instruments, USA) gemessen. Die X-Achse steht für die logarithmische Skala der Teilchengrößen in nm. Die Y-Achse zeigt den prozentualen Anteil.

Die Fig. 5 zeigt ein IR-Spektrum des beschriebenen Konjugats. Aus dem Spektrum ist Folgendes erkennbar: ein starker breiter Streifen mit einem Maximum bei 3400 cm⁻¹, ein starkes Signal bei einer Frequenz von 1621 cm⁻¹, sechs mittelstarke Streifen bei 2924, 2881, 1383, 1306, 1212 und 1154 cm⁻¹ und ein schwaches Kennsignal bei 504 cm⁻¹. Das Spektrum hat seine Höchstwerte bei 1306, 1212 und 1154 cm⁻¹, welche den Höchstwerten der Ausgangsaminosäure Glyzin entsprechen, die infolge der Bildung von einer kovalenten Bindung auf der Oberfläche der Nanodiamantenteilchen in den Bereich von 1440 - 1100 cm⁻¹ verschoben wurde.

Die IR-Spektren wurden mit einem Gerät FTIRS IR200 Thermonicolet (Thermo Scientific, USA) aufgenommen. Die Auflösung des Geräts ist 2 cm⁻¹ und die Scan-Menge 64. Die Einwaagen der Proben wurden zwecks Analyse mit einem KBr-Pulver vermengt und zu einer Tablette gepresst.

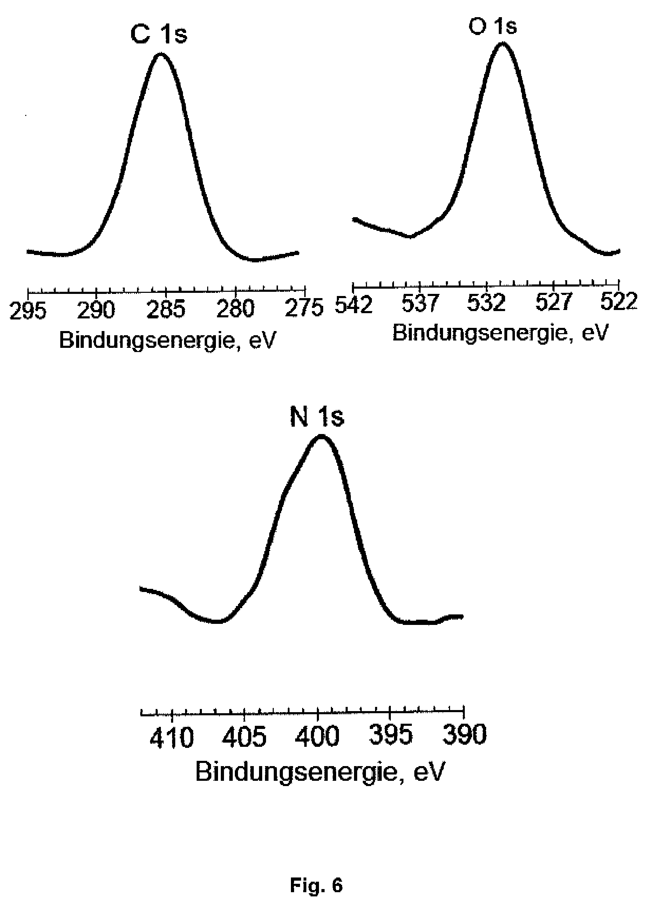

Die Fig. 6 zeigt Spektren der Röntgen-Photoelektronenspektroskopie des beschriebenen Konjugats von Nanodiamantenteilchen mit Glyzin. Die Anwendung der Röntgen-Photoelektronenspektroskopie ermöglicht es, den Charakter, den Energiezustand und die Menge der Oberflächenatome von Nanodiamantenteilchen festzustellen und so gut wie alle chemischen Elemente bis auf Wasserstoff und Helium zu ermitteln [5]. Die Oberfläche des beschriebenen Konjugats von Nanodiamantenteilchen mit Glyzin wurde mit Hilfe eines Geräts LAS-3000 (Riber, Frankreich) erforscht. Das Gerät war mit einem halbkugelförmigen Analysengerät OPX-150 ausgerüstet. Die Fotoelektronen wurden mittels nichtmonochromatischer Röntgenstrahlung einer Alu-Anode (AlK_{α} = 1486,6 eV) bei einer Röhrenspannung von 12 kV und einem Emissionsstrom von 20 mA angeregt. Die Kalibrierung der Photoelektronen-Spitzenwerte wurde anhand der Kohlenstoffkurve C 1 s mit einer Bindungsenergie (E_{Bind}) von 285 eV vorgenommen. Das Vakuum in der Arbeitskammer betrug 6,7 10⁻⁸ Pa. Um ein Hochvakuum aufzubauen, wurde eine Ionenpumpe verwendet.

Die Elementenzusammensetzung der Oberfläche des beschriebenen Konjugats von Nanodiamantenteilchen mit Glyzin nach Angaben der Röntgen- Photoelektronenspektroskopie ist in Tabelle 1 angeführt.

**Tabelle 1**

| Elementenzusammensetzung und Bindungsenergien der Oberflächenatome des beschriebenen Konjugats von Nanodiamantenteilchen mit Glyzin | | | |
|---|---|---|---|
| Eigenschaft | Chemische Elemente | | |
| | C | O | N |
| Atom %, | 77,5-94,5 | 4-14 | 1,5-8,5 |
| Bindungsenergien, eV | 285,2±0,5 | 530,7±0,5 | 399,8±0,5 |

Aus der Tabelle 1 ist ersichtlich, dass das beschriebene Konjugat von Nanodiamantenteilchen mit Glyzin keine Fluoratome sowie keine Atome anderer Halogene enthält, deren Menge die Gerätfehler (0,1 % (at.)) überschreitet. Das liegt daran, dass alle Chloratome während des Herstellungsvorgangs des Konjugats von Nanodiamantenteilchen mit Glyzin (Fig. 1) durch Glyzinmoleküle ersetzt werden und die Oberfläche der Nanodiamantenteilchen als HCl-Moleküle verlassen.

Beim beanspruchten Herstellungsverfahren werden chlormodifizierte Nanodiamantenteilchen verwendet. Diese werden wie folgt produziert: Die Nanodiamantenteilchen werden in einer Wasserstaffgasströmung H₂ bei einem Durchsatz von 2 - 3 L/Stunde bei einer Temperatur von 500 - 1200° C im Laufe von 1 - 8 Stunden ausgeglüht. Dann werden die ausgeglühten Nanodiamantenteilchen einer Flüssigphasenchlorierung mit molekularem Chlor ausgesetzt. Das Chlor wird dafür zuerst infolge einer Reaktion zwischen K₂Cr₂O₇ (bzw. KMnO₄) und Salzsäure produziert und dann in CCl₄ aufgelöst, bis eine Konzentration von 3 - 5% (Gew.) erreicht ist. Die Chlorierungsreaktion wird unter photochemischer Wirkung des sichtbaren Lichts im Laufe von 36 - 60 Stunden bei einer Temperatur von 50 - 70° C durchgeführt. Nachher wird die Probe mit CCl₄ gewaschen. Die Aufschwemmung wird bei einer Geschwindigkeit von 6000 U/min. zentrifugiert. Anschließend erfolgt eine Vakuumtrocknung bis zu einem konstanten Gewicht.

Das Verfahren zur Herstellung des Konjugats von Nanodiamantenteilchen mit Glyzin ist nachfolgend ausführlich beschrieben:
Die Aufschwemmung der chlorierten Nanodiamantenteilchen wird in einem organischen, wasserorganischen Polar-Lösungsmittel oder in Wasser vorbereitet und dann mit Glyzin in Form von Aminoessigsäure NH₂CH₂COOH mit Zugabe von tertiärem Amin ergänzt. Als organisches Lösungsmittel sollten hauptsächlich solche Lösungsmittel verwendet werden, die Glyzin auflösen können, z. B. Pyridin oder niedere aliphatische Alkohole. Das hergestellte Gemisch wird mit Ultraschall (50 W) im Laufe von 5 - 60 Min. behandelt und unter ständigem Umrühren und bei einer Temperatur von 50 - 80° C innerhalb von 12 - 48 Stunden gehalten. Das erzeugte Produkt wird mit Äthanol gewaschen und dann zentrifugiert. Der Absatz wird im Vakuum bei 70° C über die ganze Nacht getrocknet.

Somit ermöglicht das erfindungsgemäße Verfahren zur Herstellung des Konjugats von Nanodiamantenteilchen mit Glyzin, einen gefährlichen, umständlichen und teuren Vorgang der Gasphasenfluorierung durch einen mehr zugänglichen, sicheren und wesentlich kostengünstigeren Vorgang der Flüssigphasenchlorierung zu ersetzen. Das teure Glyzin-Derivat - Glyzin-Äthyläther-Hydrochlorid - wird durch eine billigere Aminosäure Glyzin ersetzt.

Das neue Konjugat von Nanodiamantenteilchen mit Glyzin enthält keine Fluoratome. Seine Partikelfeinheit in der Suspension ist 6 - 12mal höher.

Das hergestellte Konjugat von Nanodiamantenteilchen mit Glyzin kann in der Medizin als ein System zur Förderung von Aminosäure Glyzin in den Körper angewendet werden. Zu diesem Zweck wird die Zusammenwirkung des hergestellten Konjugats mit Zellkulturen nach zellbiologischen Verfahren mit Hilfe eines Elektronenmikroskops erforscht.

Kurze Beschreibung der Grafiken:
- Fig. 1: eine schematische Darstellung der Herstellung des Konjugats von Nanodiamantenteilchen mit Glyzin,
- Fig. 4: die Teilchengrößenverteilung des beschriebenen Konjugats von Nanodiamantenteilchen mit Glyzin in der Suspension,
- Fig. 5: das IR-Spektrum des beschriebenen Konjugats von Nanodiamantenteilchen mit Glyzin,
- Fig. 6: die Elemente C 1s, O 1 s, N 1 s in der Röntgen-Photoelektronenspektroskopie-Spektren der Oberfläche des beschriebenen Konjugats von Nanodiamantenteilchen mit Glyzin,
- Fig. 7: die IR-Spektren der Mischungen von Nanodiamantenteilchen mit Glyzin zur Darstellung von Eichlinien I, II, III und die Spektren der Mischungen mit ihrem jeweiligen Glyzingehalt 1 : 1 ,75 : 2,5 (die Kennspitzenwerte sind eingerahmt),
- Fig. 8: die Eichlinien für jeden Kennstreifen des IR-Spektrums der Mischung von Nanodiamantenteilchen mit Glyzin; a, b, c sind jeweils Eichlinien für die Streifen 1407, 1332 und 504 cm⁻¹,

Die Erfindung wird am folgenden Beispiel beschrieben:

### Beispiel

300 mg der Ausgangs-Nanodiamantenteilchen werden in einer Wasserstoffgasströmung H₂ mit einem Durchsatz von 3,0 L/Stunde bei einer Temperatur von 1000° C im Laufe von 6 Stunden ausgeglüht. Molekulares Chlor wird in 40 mL CCl₄ bis zu einer Konzentration von 6% (Gew.) aufgelöst. Dann werden die ausgeglühten Nanodiamantenteilchen nach dem Verfahren einer Flüssigphasenchlorierung mit molekularem Chlor Cl₂ chloriert. Die Chlorierungsreaktion wird unter photochemischer Wirkung des sichtbaren Lichts im Laufe von 60 Stunden bei 60° C durchgeführt. Danach wird die Probe mit CCl₄ gewaschen, wonach die Suspension bei einer Geschwindigkeit von 6000 U/min. zentrifugiert wird. Anschließend erfolgt eine Trocknung unter einem Druck von 0,1 mm Hg, bis ein konstantes Gewicht erreicht ist. Dann wird auf der Grundlage der chlorierten Nanodiamantenteilchen eine Aufschwemmung vorgenommen, in der 40 mL Alkohol-Wasser-Gemisch (Methanol: Wasser =1:1) verwendet wird. Die Aufschwemmung wird mit 300 mg Glyzin in Form von freier Aminosäure NH₂CH₂COOH mit Zugabe von 1 mL Triäthylamin ergänzt. Das hergestellte Gemisch wird mit Ultraschall (50 W) 60 Minuten lang behandelt und unter ständigem Umrühren bei 65° C im Laufe von 30 Stunden gehalten. Das erzeugte Produkt wird mit einer großen Menge Äthanol gewaschen, zentrifugiert und im Vakuum bei 70° C über die ganze Nacht getrocknet. Die Restfeuchte des Produkts beträgt 2,2 %. Die Zielproduktausbeute beträgt 279 mg (93 %).

Das erzeugte Produkt stellt ein dunkelgraues, blau angefärbtes ultradisperses Pulver mit Primärteilchengrößen im Bereich von 2 - 10 nm dar. Die Teilchen haben eine bis 1 nm starke Oberflächenhülle. Das Produkt ist durch folgendes IR-Spektrum gekennzeichnet: ein starker breiter Streifen bei 3400 cm⁻¹, ein starkes Signal bei einer Frequenz von 1621 cm⁻¹, sechs mittelstarke Streifen bei 2924, 2881, 1383, 1306, 1212 und 1154 cm⁻¹ und ein schwaches Kennsignal bei 504 cm⁻¹. Die Teilchengröße in der Suspension des hergestellten Produkts betrug 25 nm. Die Elementenzusammensetzung der Produktoberfläche ist in Tabelle 3 enthalten.

**Tabelle 3**

| Angaben der Röntgen-Photoelektronenspektroskopie zum hergestellten Produkt | | | |
|---|---|---|---|
| Eigenschaft | Chemische Elemente | | |
| | C | O | N |
| Atom %, | 80,1±0,1 | 11,5±0,1 | 8,4±0,1 |
| Bindungsenergien, eV | 285,2±0,5 | 530,7±0,5 | 399,6±0,5 |

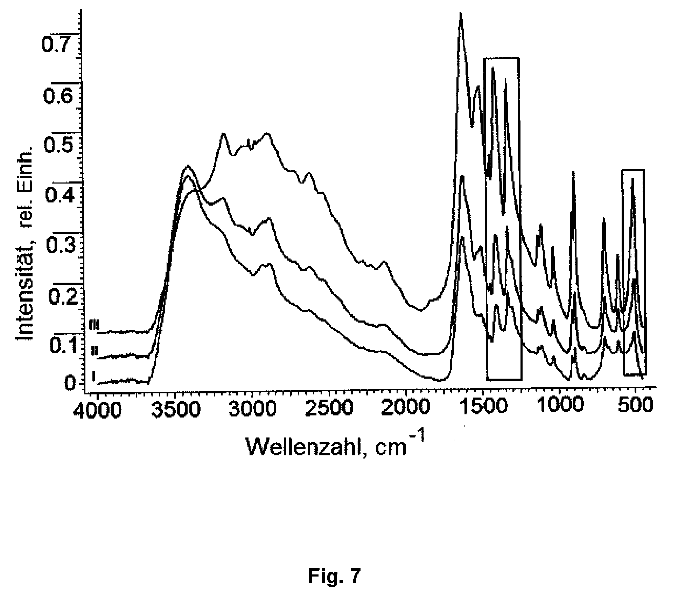

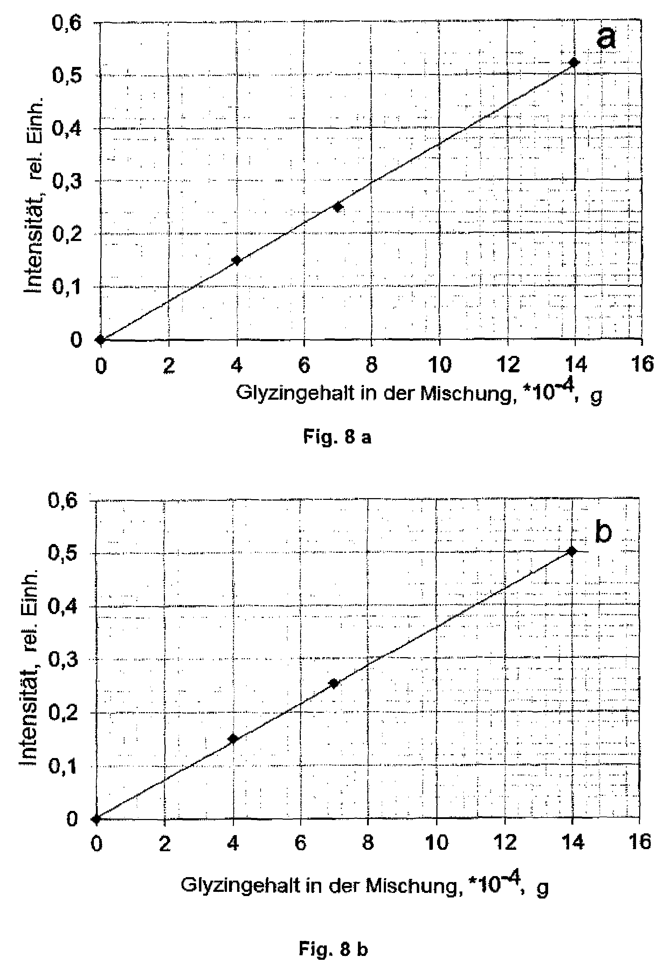

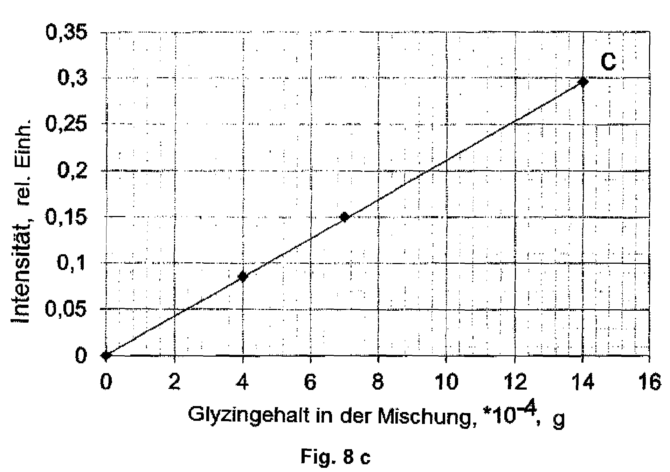

Um den Massenanteil von Glyzin im hergestellten Konjugat zu bestimmen, werden drei Mischungen von Nanodiamantenteilchen mit Glyzin mit folgendem Glyzin-Verhältnis vorbereitet: 1 : 1,75 : 3,5. Jeder Mischung wird je eine Einwaage (0,0035 g) entnommen und sorgfältig mit je 0,090 g KBr in einem Pulvermörser gerieben. 0,070 g der hergestellten Mischung werden zu einer Tablette gepresst und ihr IR-Spektrum wird aufgenommen (Fig. 7). Die Kennstreifen werden jeweils bei 1407, 1332 und 504 cm⁻¹ gewählt. Für jeden Kennstreifen werden Eichlinien erstellt (Fig. 8). Die Streifen intensität der entsprechenden Kennstreifen im IR-Spektrum der hergestellten 0,0035 g schweren Probe des Konjugats von Nanodiamantenteilchen mit Glyzin beträgt jeweils 0,23, 0,22 und 0,10 rel. Einh. Aufgrund der Eichlinien a, b, c (Fig. 8) wird der Glyzingehalt in der Probe bestimmt. Er beträgt 0,00057 ± 8.10⁻⁵ g in der Probe. Folglich ist der Massenanteil von Glyzin in der Probe-Einwaage 21 ± 3 % (Gew.).

Die Behandlung der Suspension mit Ultraschall im Laufe von 30 - 60 Minuten, das Halten bei einer Temperatur von 70 - 80° C im Laufe von 30 - 48 Stunden sowie die Nutzung von Pyridin oder niederen aliphatischen Alkoholen als Polar-Lösungsmittel ergibt ein Konjugat mit ähnlichen Eigenschaften und einem Glyzingehalt im Bereich von 13 - 21 % (Gew.).

Die Behandlung der Suspension mit Ultraschall im Laufe von 5 - 30 Minuten, das Halten bei einer Temperatur von 50 - 70° C im Laufe von 12 - 30 Stunden sowie die Verwendung von Alkohol-Wasser-Gemisch oder Wasser als Polar-Lösungsmittel ergeben ein Konjugat mit ähnlichen Eigenschaften und einem Glyzingehalt im Bereich von 2 - 14 % (Gew.).

Das hergestellte Konjugat von Nanodiamantenteilchen mit Glyzin wird zur Förderung von Glyzin in den Körper eingesetzt. Die Eindringung des Konjugats von Nanodiamantenteilchen mit Glyzin in den Körper wird durch Forschungen der Zusammenwirkung zwischen dem Nanodiamanten-Konjugat mit Glyzin und der Lymphoblast-Zellkultur MOLT-4 mit Hilfe eines elektronischen Mikroskops nachgewiesen. Die Inkubationszeit beträgt dabei 8 Stunden. Es ist ersichtlich, dass unter der Konjugatwirkung Invaginationen der Zellmembran der Lymphoblastzelle zustande kommen. Diese vertiefen sich allmählich und führen dazu, dass die Zelle das Konjugat von Nanodiamantenteilchen mit Glyzin völlig aufnimmt.

Die Schnitte der mit dem hergestellten Konjugat inkubierten Zellen wurden mit Hilfe von Ultramikrotom Leica Ultracut UCT (Leica, Deutschland) gefertigt. Die Mikroaufnahmen der Zellenschnitte wurden mit einem Durchstrahlungselektronen-ikroskop JEM 1230 (JEOL, Japan) gemacht.

### Schriftennachweis

1. Patent US 2005/0158549 A1, 21.07.2005.
2. Y. Liu, Zh. Gu, J.L. Margrave, V.N. Khabashesku. Functionalization of Nanoscale Diamond Powder: Fluoro-, Alkyl-, Amino-, and Amino Acid- Nanodiamond Derivatives // Chem. Mater. 2004. V.16. P. 3924-3930.
3. Russisches Sammelbuch für Arbeitsschutz. 3 Bände. 2. aktualisierte Auflage. Band 3. M.: Verlag NZ ENAS. 2007. S. 181.
4. T.I. Shalina, L.S. Vassilieva. Allgemeine Fragen zur giftigen Fluorwirkung // Sibirische Medizinische Zeitschrift. 2009. Heft 5. S. 5-9.
5. Elektronen- und Ionenspektroskopie der festen Körper / Herausgegeben von L.I. Firmens et al. - M.: Mir. - 1981, - S. 195-232.

## Patentansprüche

1. Konjugat von Nanodiamanten mit Glyzin zur Förderung von Glyzin in den Körper in Form von glyzinmodifizierten Nanodiamantenteilchen mit einer Korngröße von 2 - 10 nm und mit einem Glyzingehalt bis zu 21 ± 3 % (Gew.), wobei Glyzin ein Bestandteil einer bis zu 1 nm starken Oberflächenhülle der Nanodiamantenteilchen ist **und keine Fluoratome auf seiner Oberfläche enthält.**

2. Konjugat von Nanodiamanten mit Glyzin zur Förderung von Glyzin in den Körper in Form von glyzinmodifizierten Nanodiamantenteilchen nach Anspruch 1, **dadurch gekennzeichnet,**
**dass**, **wenn als wässrige Suspension vorliegend, die Aggregatgröße von 25 - 50 nm beträgt.**

3. Verfahren zur Herstellung von Konjugat von Nanodiamanten mit Glyzin nach Anspruch 1 oder 2,
**dadurch gekennzeichnet,**
**dass** chlormodifizierte Nanodiamantenteilchen mit einer Korngröße von 2 - 10 nm in einem Polar-Lösungsmittel aufgelöst werden, so dass eine Aufschwemmung gebildet wird,
**dass** danach der Aufschwemmung tertiäres Amin und Glyzin zugesetzt werden und
**dass** das hergestellte Gemisch anschließend mit Ultraschall behandelt und dann bei 50 °C gehalten, zentrifugiert, mit Lösungsmittel gewaschen und getrocknet wird.

4. Verfahren nach Anspruch 3,
**dadurch gekennzeichnet,**
**dass** die Ultraschallbehandlung im Laufe von 5 - 60 Minuten und das Halten bei 50 °C im Laufe von 12 - 48 Stunden vorgenommen werden.

5. Verfahren nach Anspruch 3 oder 4,
**dadurch gekennzeichnet,**
**dass** als tertiäres Amin Triethylamin und als Polar-Lösungsmittel Pyridin, ein niederer aliphatischer Alkohol, ein Alkohol-Wasser-Gemisch oder Wasser verwendet werden.

6. Konjugat von Nanodiamanten mit Glyzin **nach Anspruch 1 oder 2 zur Verwendung in der Medizin.**

## Claims

1. Conjugate of nanodiamonds with glycine to promote glycine in the body in the form of glycine-modified nanodiamond particles having a particle size 2-10 nm and a glycine content up to 21 ± 3% (wt.), wherein the glycine is a component of a surface shell of the nanodiamond particles up to 1 nm thick and contains no fluorine atoms on its surface.

2. Conjugate of nanodiamonds with glycine to promote glycine in the body in the form of glycine-modified nanodiamond particles according to claim 1, **characterised in that** when an aqueous suspension is present, the aggregate size is 25 - 50 nm.

3. Method for producing conjugate of nanodiamonds with glycine according to claim 1 or 2, **characterised in that**,
- chlorine-modified nanodiamond particles with a grain size of 2 - 10 nm are dissolved in a polar solvent to form a suspension,
- tertiary amine and glycine are then added to the slurry and
- the mixture so produced is then treated with ultrasound and then maintained at 50 °C, centrifuged, washed with solvent and dried.

4. Method according to claim 3, **characterised in that**
- the ultrasound treatment is effected for 5 - 60 minutes, while the temperature maintenance at 50 °C is effected for 12 - 48 hours.

5. Method according to claim 3 or 4, **characterised in that** triethylamine is used as a tertiary amine, while pyridine, a lower aliphatic alcohol, an alcohol-water mixture or water is used as a polar solvent.

6. Conjugate of nanodiamonds with glycine according to claim 1 or 2 for use in medicine.

## Revendications

1. Conjugué de nanodiamants et de glycine pour le transport de glycine dans le corps, sous forme de particules de nanodiamants modifiées par de la glycine, ayant une granulométrie de 2 à 10 nm et une teneur en glycine allant jusqu'à 21 ± 3 % (en poids), la glycine étant un constituant d'une enveloppe superficielle, ayant une épaisseur allant jusqu'à 1 nm, des particules de nanodiamants, et ne contenant aucun atome de fluor sur sa surface.

2. Conjugué de nanodiamants et de glycine pour le transport de glycine dans le corps, sous forme de particules de nanodiamants modifiées par de la glycine, selon la revendication 1, **caractérisé en ce que**, quand il se présente sous forme d'une suspension aqueuse, la grosseur des agrégats est de 25 à 50 nm.

3. Procédé de fabrication d'un conjugué de nanodiamants et de glycine selon la revendication 1 ou 2, **caractérisé en ce que** l'on dissout des particules de nanodiamants modifiées par du chlore, ayant une granulométrie de 2 à 10 nm, dans un solvant polaire, de façon qu'il se forme une suspension ; que la suspension est ensuite additionnée d'une amine tertiaire et de glycine ; et que le mélange préparé est ensuite traité par des ultrasons puis est maintenu à 50°C, centrifugé, lavé avec un solvant et séché.

4. Procédé selon la revendication 3, **caractérisé en ce que** le traitement aux ultrasons est mis en oeuvre pendant 5 à 60 minutes, et le maintien à 50°C est réalisé pendant 12 à 48 heures.

5. Procédé selon la revendication 3 ou 4, **caractérisé en ce qu'**on utilise en tant qu'amine tertiaire de la triéthylamine, et en tant que solvant polaire de la pyridine, un alcool aliphatique inférieur ou un mélange alcool-eau, ou de l'eau.

6. Conjugué de nanodiamants et de glycine selon la revendication 1 ou 2, pour une utilisation en médecine.
